# EUROPEAN PATENT APPLICATION

(11) **EP 3 231 449 A1**
(43) Date of publication of application: **18.10.2017**
(21) Application number: 16425032.6
(22) Date of filing: 12.04.2016
(51) Int. Cl.: A61L 9/12, B60H 3/00

(54) **DEODORANT DEVICE FOR PASSENGER COMPARTMENTS OF MOTOR VEHICLES**

(71) Applicant: RE.LE.VI. S.P.A., 46040 Rodigo (Mantova) (IT)
(72) Inventor: Venini, Roberto Cherubino, I - 46040 Rodigo (MN) (IT)
(74) Representative: Perani, Aurelio

(57) **Abstract**

Air freshener device for automotive vehicle compartments, comprising a support (1) having means (2) for removable connection thereof to the dash panel of a motor vehicle, a fragrance-containing capsule (15) adapted to be associated with said support, said capsule having a fragrance-containing compartment (16) and an opening in said compartment, which is covered with an osmotic membrane (24) for fragrance release. The device has a cavity (5), with a closed bottom (6) and an open top end (7), a first vertical wall (8) externally equipped with said means (2) for removable connection to the dash panel, a second vertical wall (10) opposite to the first wall, as well as walls (11, 12) that enclose the cavity (5) at its sides, said second wall (10) having an opening (14) which is designed to be in register with said opening (23) with the osmotic membrane (24) of the compartment (16) of the capsule (15) when the latter is associated with the support and placed in its cavity (5).

Should the osmotic membrane be damaged, the liquid of fragrant liquid leaking out of the capsule would flow into said cavity (5) of the device, whereby damages to the dash panel of the automotive vehicle having the device mounted thereto would be avoided.

## Description

The present invention relates to an air freshener device for automotive vehicle passenger compartments, comprising a support having means for removable connection thereof to the dash panel of the motor vehicle, a fragrance-containing capsule adapted to be associated with said support, said capsule having a fragrance-containing compartment and an opening in said compartment, which is covered with an osmotic membrane for fragrance release.

Air freshener devices as set forth above are known in the art.

They are typically attached to the air baffles of the air outlets in the passenger compartment of the automotive vehicle, which are situated on the dash board, and as the liquid fragrance-containing capsule is depleted, it is replaced with a new one while the support is still used.

In spite of their structural simplicity, these air freshener devices are affected by certain technical drawbacks associated with the replacement of the depleted capsule with a new one.

During replacement, when attempting to find the proper position for the new capsule in the support, the osmotic membrane may be broken and the fragrant liquid may leak out, thereby damaging or at least fouling the dash panel of the vehicle.

The object of the present invention is to limit the drawbacks caused when the osmotic membrane of the fragrant liquid-containing capsule breaks, by providing a technical solution for capsule support and for the means for capsule replacement in the support when depleted, that reduces the risk of deforming the capsule and breaking its osmotic membrane or, should it be actually broken, that prevents the fragrant liquid from leaking out and damaging the dash panel of the automotive vehicle upon which the air freshener device is mounted.

These and other objects, as better explained hereafter, are fulfilled by an air freshener device for automotive vehicles as defined in the accompanying claim 1.

The invention will be now described in greater detail with reference to a preferred embodiment thereof, which is by way of example and without limitation in the annexed drawings, in which:

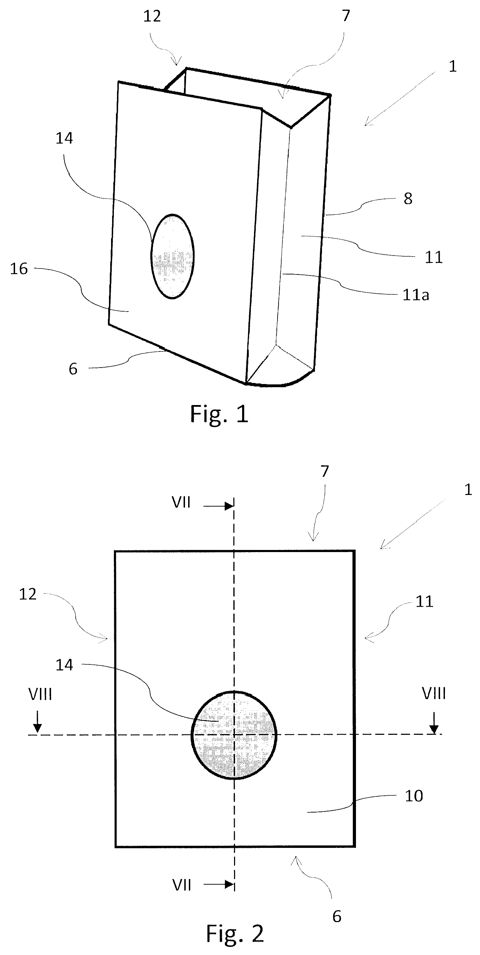

- Figure 1 shows a schematic perspective view of the air freshener device of the invention;
- Figure 2 shows a front view of the device of Figure 1;

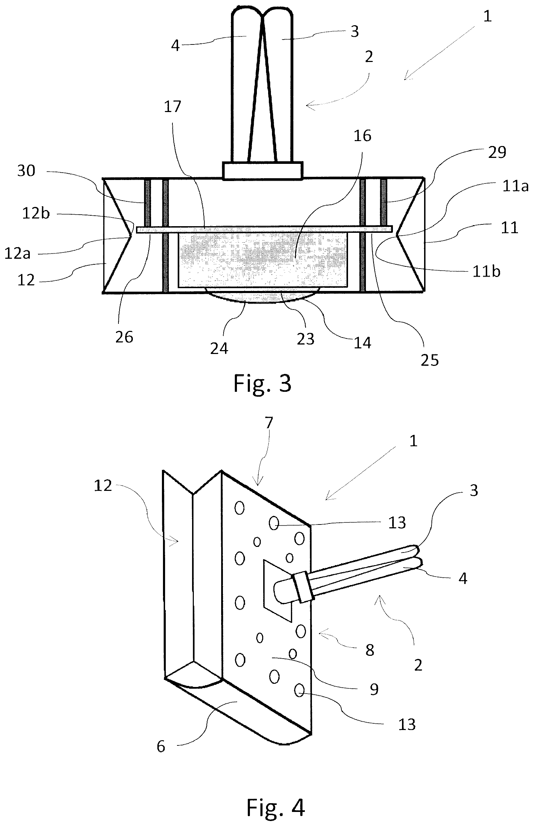

- Figure 3 shows a partially sectional side view of the device of Figures 1 and 2, with the capsule inserted therein;
- Figure 4 shows a side and rear view of the device of the previous figures;
- Figure 5 is a plan view of the fragrant liquid-containing capsule of the device of the invention;
- Figure 6 shows a side view of the capsule of Figure 5;
- Figure 7 shows a sectional view of the device of the invention, as taken along the line VII-VII of Figure 2;
- Figure 8 shows a sectional view of the device of the invention, as taken along the line VIII-VIII of Figure 3.

Referring to the aforementioned figures, the device of the invention comprises a support, generally designated by numeral 1, which has means, generally designated by numeral 2, for removable connection thereof to the dash panel of an automotive vehicle, both of which are not shown because they have a conventional construction.

These means 2 are preferably composed of a pair of elastic tines 3 and 4 which can fit, for example onto a baffle, not shown, of an air outlet, as conventionally provided on the dash panel.

The support 1 comprises a cavity 5 with a closed bottom 6 and an open top end 7. This cavity is formed by a first wall 8, with the tines 3 and 4 extending from its outer surface 9, a second wall 10 opposite to the wall 8, and the walls 11 and 12 which close said cavity 5 at its sides.

Holes or slots 13 are preferably formed in the wall 8 for ventilating, when needed, the interior of the cavity 5 of the device.

An opening 14, preferably having a circular shape, is formed in the wall 10, with the osmotic membrane being in register therewith, for releasing the fragrant material in a conventional manner, as best shown hereinafter.

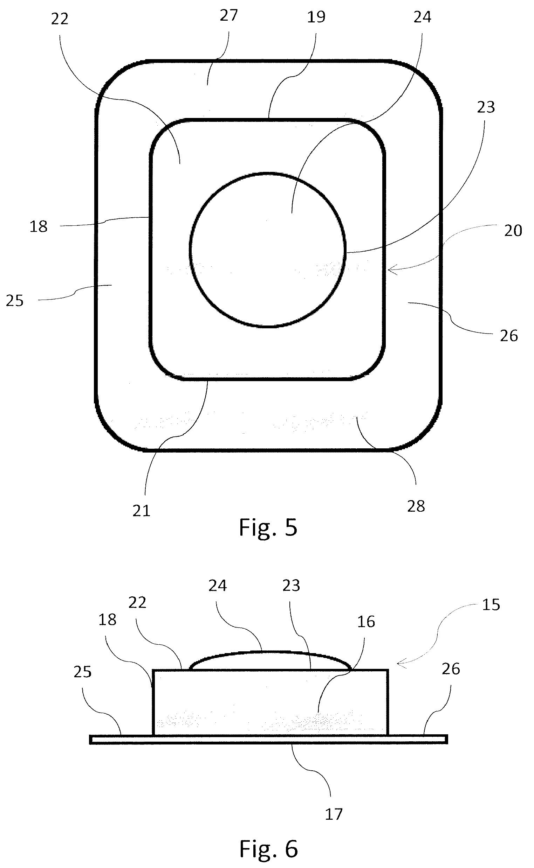

Referring now to Figures 5 and 6, the capsule, generally referenced 15, which is designed to be associated with the support 1 of the device, is shown to comprise a compartment 16 with the fragrant or scenting liquid housed therein.

The compartment 16, which has a four-sided shape in the illustrated embodiment, is closed by a bottom 17, preferably made of a rigid or semirigid material, side walls 18, 19, 20 and 21 and a top wall 22.

In such top wall 22, the capsule has an opening 23, which is covered by a conventional osmotic membrane , referenced 24, which is designed to be covered by a conventional removable protective film when the capsule is not in operation in the device.

The bottom 17 extends beyond the contour of the compartment 16 defined by the walls 18, 19, 20 and 21, with a flat rim, also having a four-sided shape, whose opposed and parallel sides are referenced 25, 26 and 27, 28.

The position of the opening 23 on the top wall 22 of the compartment 16 is preferably centered at least with respect to the linear dimension between the opposite flat rims 27 and 28, for the capsule 15 to be able to fit into the cavity 5 of the device 1 on the side of either the rim 27 or the rim 28, and allow the osmotic membrane 24 to be always positioned in register with the opening 14 of the wall 10 of the device, as explained hereinafter.

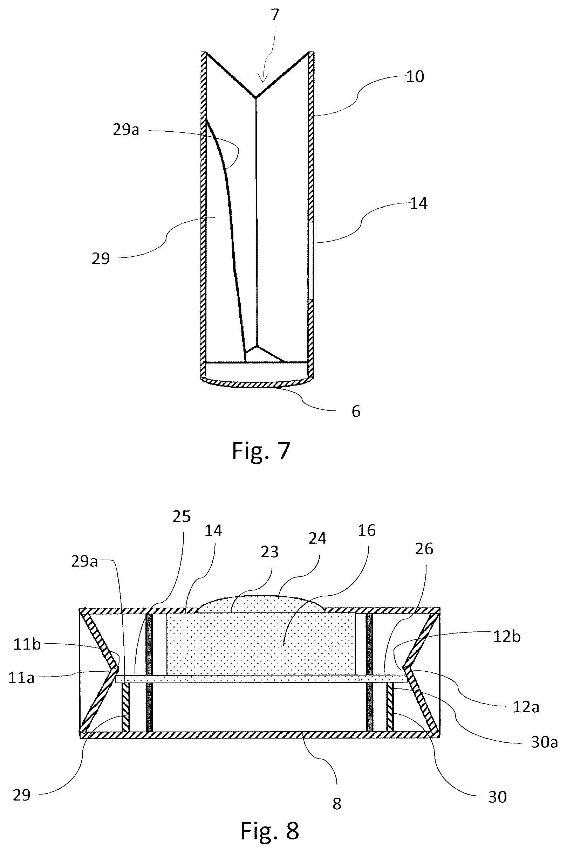

Referring to Figures 7 and 8, the wall 8 of the support 1 is shown to have a pair of parallel ribs, referenced 29 and 30, which extend from an area proximate to the open top 7 of the cavity 5 to an area proximate to the closed bottom 6 of the same cavity 5.

The respective free edges 29a, and 30a of the ribs progressively slope toward the opposite wall 10 in the direction of the closed bottom 6 of the cavity 5.

These parallel ribs 29 and 30 are at such a distance that, when the capsule 15 is placed in the cavity 5, the opposed flat rims 25 and 26 of the four-sided contour of the capsule compartment 16 lie over the free edges 29a and 30a.

Particularly referring to Figure 8, each of the walls 11 and 12 that enclose the cavity 5 at its sides has a fold 11a, 12a respectively, which creates a projection 11b, 12b in the cavity 5, such projection being close to the free edge of the ribs 29 and 30 near the bottom 6 and lying, for a given length, over the flat rims 25 and 26 of the four-sided contour of the compartment 16 of the capsule 15, thereby acting as a positioning aid therefor, when the capsule is inserted in the cavity 5, for the opening 23 with the osmotic membrane 24 thereof to be and remain in register with the opening 14 of the wall 10.

It may be appreciated from the above that the ribs 29 and 30, in combination with the opposed rims 25 and 25 of the capsule compartment, afford guided insertion of the capsule into the cavity 5 during replacement, until it reaches its position in register with the opening 14 of the support, thereby avoiding any distortion and consequent rupture of the capsule.

Nevertheless, should any damage occur to the capsule in its osmotic membrane due to improper insertion thereof, any fragrant liquid leakage occurring when the device is in operation on the dash panel of an automotive vehicle, would cause no damage, because it would be collected in the closed-bottom cavity 5 of the device.

The device of the invention as described above can be obviously provided with structural variants and changes within the inventive scope, as claimed below.

## Claims

1. An air freshener device for automotive vehicle passenger compartments comprising a support (1) having means (2) for removable connection to the dash panel of an automotive vehicle, a fragrance containing capsule (15) adapted to be associated with said support, said capsule having a fragrance-containing compartment and an opening (23) in said compartment, which is covered with an osmotic membrane (24) for fragrance release, **characterized in that** said support has a cavity (5) with a closed bottom (6) and an open top end (7), a first vertical wall (8) externally equipped with said means (2) for removable connection to the dash panel, a second vertical wall (10) opposite to the first wall, as well as walls (11, 12) that enclose the cavity (5) at its sides, said second wall (10) having an opening (14), which is designed to be in register with said opening (23) with the osmotic membrane (24) of the compartment (16) of the capsule (15) when the latter is associated with the support and placed in its cavity (5).

2. A device as claimed in claim 1, **characterized in that** the cavity (5) of said support (1) and said capsule (15) have respective positioning means (25, 26, 29, 30), which are adapted to allow said opening (23) with the osmotic membrane (24) of the fragrance-containing compartment (16) of the capsule (15) to be in register with the opening of said second wall (10) of the cavity (5) of the support when the capsule is inserted in said cavity.

3. A device as claimed in claim 1 and 2, wherein said means for positioning the capsule in the cavity of the support comprise a four-sided contour for the fragrance-containing compartment (16) of the capsule, at least one pair of flat rims (25, 26) projecting out of two opposed sides of said four-sided contour of the compartment (16), a pair of ribs (29, 30) projecting out of the inner surface of said first wall of the cavity, opposed to that (10) with the opening (14), said ribs extending parallel to each other from an area proximate to said open top end (7) of the cavity to an area proximate to the closed bottom (6) of such cavity (5) and being at such a distance from each other that said opposed flat rims (25, 26) of the four-sided contour of the compartment (16) of the capsule, when the latter is placed in the cavity (5) of the support, lie over the free edges (29a, 30a) of the ribs (29, 30).

4. A device as claimed in claim 3, wherein the free edge (29a, 30a) of each of said ribs projecting out of the inner surface of said first wall (8) of the cavity (5) of the support progressively slopes toward the opposite wall (10) with the opening (14), in the direction of the closed bottom (6) of said cavity (5) of the support.

5. A device as claimed in claims 1 to 4, **characterized in that** each of said walls (11, 12) that enclose the cavity (5) of the support at its sides has a projection (11b. 12b) facing the interior of the cavity (5) and overlying the free edge (29a, 30a) of the rib of the pair of ribs that faces the wall, said projection being located proximate to the bottom (6) of the cavity (5).

6. A device as claimed in claim 5, wherein said projection (11b, 12b) of each of said walls (11, 12) which enclose the cavity (5) at its sides consists of a portion of a fold (11a, 12a) facing the interior of the cavity (5) and formed in each of said side walls.

7. A device as claimed in any of claims 1 to 6, wherein said first wall (8) externally equipped with said means (2) for removable connection to the dash panel, has a plurality of openings (13) communicating with the cavity (5) of the support.
